Europäisches Patentamt

European Patent Office

Office européen des brevets

(19)

(11) Numéro de publication: **0 202 160**
**A2**

(12) **DEMANDE DE BREVET EUROPEEN**

(21) Numéro de dépôt: 86400985.7

(22) Date de dépôt: 07.05.86

(51) Int. Cl.⁴: **A61B 5/10** , A61N 1/36 ,
A61N 1/08

(30) Priorité: 10.05.85 BE 215002

(43) Date de publication de la demande:
**20.11.86 Bulletin 86/47**

(84) Etats contractants désignés:
**AT CH DE FR GB IT LI LU NL SE**

(71) Demandeur: **Peucelle, Guy Paul Marcel**
**55 rue de Mareil**
**F-78100 Saint Germain en Laye(FR)**
Demandeur: **Klotz, Antoine Olivier**
**1 coeur de Maule**
**F-78580 Maule(FR)**

(72) Inventeur: **Peucelle, Guy Paul Marcel**
**55 rue de Mareil**
**F-78100 Saint Germain en Laye(FR)**
Inventeur: **Klotz, Antoine Olivier**
**1 coeur de Maule**
**F-78580 Maule(FR)**

(54) **Appareil électronique à programmation destiné à l'acquisition de l'information relative à la contraction des muscles.**

(57) L'invention concerne un appareil électronique à programmation, destiné à l'acquisition de l'information relative à la contraction des muscles, à l'état hémodynamique et à la stimulation du retour veineux, notamment des muscles lisses du tissu vasculaire, et méthode d'utilisation de cet appareil pour le traitement des maladies liées directement ou indirectement au mauvais fonctionnement ou aux déficiences des systèmes vasculaires et neurovasculaires.

EP 0 202 160 A2

# APPAREIL ELECTRONIQUE A PROGRAMMATION, DESTINE A L'ACQUISITION DE L'INFORMATION RELATIVE A LA CONTRACTION DES MUSCLES.

La présente invention a pour objet un appareil électronique à programmation, destiné à l'acquisition de l'information relative à la contraction des muscles, incluant notamment des muscles lisses du tissu vasculaire, à l'état hémodynamique et à la stimulation du retour veineux. Elle vise également une méthode d'utilisation de cet appareil pour le traitement des maladies liées, directement ou indirectement, au mauvais fonctionnement at aux déficiences des systèmes vasculaires et neurovasculaires et trophiques. La présente invention se rapporte également à un procédé d'exploration fonctionnelle des tissus vasculaires et neurovasculaires. Elle se rapporte également à un système de rééducation des sphincters urétral et anal.

Jusqu'à présent les modes de prévention des maladies dues à des déficiences des tissus vasculaires et vasculonerveux étaient essentiellement chimiothérapiques. On préconisait notamment l'usage d'anticoagulants, d'antiagrégants plaquettaires et d'autres produits associés. Bien qu'il fallait mettre oeuvre, lors de l'application de tels médicaments, de nombreux et coûteux tests biologiques, leur efficacité est loin d'être probante, tandis que leur coût direct pour les hôpitaux publics ne cesse d'augmenter annuellement, dépassant par example pour la France le chiffre de 2 milliards de Francs par an.

A la suite des travaux réalisés par les docteurs LAQUERRIERE et LOUBIER, et le docteur LEGOS, un certain nombre de recherches ont été faites, qui ont conduit à la réalisation d'appareils électriques, par example celui de Pollock destiné à stimuler le triceps sural, mais qui ne pouvait être utilisé sur le malade à l'état de veille. On peut également citer parmi les publications de l'art antérieur, le brevet allemand n° 976 354 (GRATZEL) qui se rapporte à la stimulation des muscles striés au moyen d'impulsions électriques. De tels appareils, de part leur conception non adaptée, ne permettaient pas l'acquisition de l'information relative à la contraction des muscles, en particulier des muscles lisses du tissu vasculaire et de l'effet de ces contractions sur le retour veineux.

Un objet de la présente invention est d'obvier aux inconvénients des appareils de l'art antérieur, en réalisant un appareil électronique à programmation, qui puisse, tout en ayant un effet thérapeutique certain, permettre l'acquisition de l'information relative à l'effet de la contraction des muscles, notamment des muscles striés lisses du tissu vasculaire, et ce grâce à la génération et à l'application dûment programmées de courants reproduisant des courants d'action physiologique.

Un autre objet de l'invention est de fournir un appareil électronique pouvant être mis en oeuvre pour le traitement des maladies liées directement ou indirectement, au mauvais fonctionnement et aux déficiences des systèmes vasculaires et neurovasculaires, de la circulation de retour veineux et du trophisme musculaire, incluant les sphincters urétral et anal.

Encore un objet de la présente invention est de réaliser un appareil électronique à programmation, permettant la mise en oeuvre d'un procédé d'exploration fonctionnelle des tissus vasculaires et vasculonerveux.

La présente invention a donc pour objet un appareil électronique à programmation permettant d'atteindre les buts précités et comportant des moyens permettant de générer des impulsions progressives positives unidirectionnelles, progressives négatives unidirectionnelles, ou successivement positives et négatives par trains comportant une pluralité d'impulsions alternées, ces moyens générateurs comportant, disposés de façon logique, des moyens de programmation de mise en oeuvre comportant un système de logique programmée et un système de logique de contrôle et de comparaison agencés en boucle logique, des moyens d'intégration définissant la forme du signal d'impulsions générées et des moyens d'amplification, et que ces moyens générateurs d'impulsions sont associés à au moins deux électrodes disposées en des endroits appropriés du corps, de façon à permettre aux impulsions de circuler d'au moins une première zone d'application des impulsions à au moins une seconde zone d'application desdites impulsions.

Les moyens générateurs sont associés à des électrodes disposées en des endroits appropriés des membres du sujet à traiter de façon à permettre aux impulsions de circuler alternativement d'un membre à l'autre en passant avantageusement par le petit bassin, dans le cas des membres inférieurs.

Le dispositif électronique à programmation selon la présente invention est en outre remarquable par les points suivants:

-les moyens de programmation sont constitués par un ensemble électronique constitué par un au moins un microprocesseur associé à au moins une est également doté d'un programme spécialisé permettant de tester toutes les fonctions et les circuits internes au moyen du clavier 8, des afficheurs 9 et/ou des liaisons externes 15.

Le dispositif générateur de signaux est constitué d'un intégrateur 12 associé à des moyens amplificateurs 11, eux-mêmes reliés à un système d'électrodes 14.

En outre, l'intégrateur 12 et l'amplificateur 11 peuvent être commandés par le microprocesseur 1 ou le comparateur 20 ; ceci permet de faire varier la forme et l'amplitude des impulsions de sortie.

La diversité de la forme et de l'amplitude des signaux générés permet la stimulation du muscle lisse du tissu vasculaire, ainsi que des muscles striés de l'environnement.

De la description qui précède, il est clair que l'utilisation du dispositif de la présente invention peut être mis en oeuvre de diverses façons, notamment en ce qui concerne l'utilisation par programmation manuelle et déclenchement au moyen des signaux émis à partir des paramètres détectés au niveau des tissus à traiter ou bien en programmant le traitement à l'avance au moyen de l'horloge à temps réel associée au microprocesseur 1.

Dans les services ou organisations nécessitant l'emploi régulier d'un certain nombre d'appareils, ceux-ci sont économiquement et avantageusement remplacés par un générateur central lié et distribué par un réseau de câbles, blindés ou non, à des terminaux individuels programmables ou non comprenant la partie nécessaire des fonctions décrites précédemment.

Les signaux délivrés par le dispositif stimulateur selon l'invention au niveau des tissus à fibres musculaires lisses à traiter et qui correspondent aux courants décrits par Laquerrière et Loubier et similaires à la chronaxie musculaire vasomotrice, peuvent être des impulsions progressives positives unidirectionnelles, des impulsions progressives négatives unidirectionnelles, ou bien des trains d'impulsions progressives alternées. On notera que lors de l'application de l'appareil de l'invention pour le traitement de tissus veineux, ces signaux, qui reproduisent les courants physiologiques, parcourent les fibres amyéliniques vasomotrices sympathiques qui aboutissent sur les parois et toute l'épaisseur de la veine et de l'endoveine. Ils peuvent franchir les vésicules de jonction et permettre de fermer le circuit électrique. Ces signaux présentent avantageusement les caractéristiques suivantes ;

-une constante de temps égale ou de l'ordre de 40 $\mu$s ± 200 $\mu$s;

-une durée d'extinction égale à la durée de l'établissement de l'impulsion ;

-une largeur à la base égale à 3 ms ± 30 ms ;

-une amplitude de crête réglable de 0 à 130 volts et de -130 à 0 V ;

-un courant de 0,05 mA à 2 mA ;

-une période égale à 541 ms ± 200 ms.

Il est préférable que la durée de l'impulsion ne dépasse jamais 80 % de la période d'impulsion, tandis que la tension de crête ne dépasse à aucun moment 125 % de la valeur de réglage, les variations de la tension de sortie ne produisant aucune modification de la fréquence de réglage. Dans le cas de trains d'impulsions progressives alternées, il est préférable de mettre en oeuvre des trains de 8 impulsions (-6, + 30) de caractéristiques identiques à celles énoncées précédemment et dont la période de récurrance se situe entre -8 et + 20 s, et est par exemple égale à 10 s. Il est également possible, selon l'invention, de mettre en oeuvre de très basses, moyennes ou hautes fréquences et/ou d'amplitudes dont les caractéristiques de l'enveloppe sont identiques à celles énoncées précédemment.

Les expérimentations cliniques, auxquelles a été soumis le dispositif stimulateur selon l'invention, ont donné des résultats particulièrement intéressants, notamment dans le traitement des maladies thrombovasculaires, de la trophicité musculaire et de la rééducation urétrale et anale. Le dispositif stimulateur de l'invention peut être associé à n'importe quel moyen d'exploration fonctionnelle, par exemple un dispositif DOPPLER, un compteur de radiations, un détecteur de radiations, un détecteur rhéologique, un détecteur de pléthysmographie et tout dispositif analogue mettant en évidence l'accélération du flux de retour et l'augmentation des indices de remplissage, de vidange d'activité du système veineux.

On notera également une action sur la totalité des muscles striés des membres entre lesquels sont appliquées les électrodes. Dans cette expérimentation, une seule électrode sur chaque membre suffit, cette électrode étant placée sur les pieds, le triceps sural ou en tout autre endroit, ce qui simplifie considérablement les manipulations pendant un acte chirurgical, par exemple sur un comateux, un vieillard at autre patient difficile à traiter. A l'expérimentation, les praticiens se sont également aperçus que ces courants ne brouillent pas les scopes de contrôle, notamment ceux d'un monitoring cardiaque. Par ailleurs, ces courants ne peuvent interférer avec le muscle cardiaque ni agir sur un stimulateur cardiaque (pacemaker). En outre, ces courants sont atténués et trop faibles pour provoquer l'ionisation. Ils peuvent être également utilisés sur des porteurs de prothèse métallique.

On s'est également aperçu que ces courants ne sont pas à l'origine de production d'acide lactique par les muscles striés qui les stimulent, pas plus qu'ils ne tétanisent lesdits muscles. On a également mis en évidence le fait que ces courants économisent la consommation de produits azotés, donc le catabolisme musculaire. Cette détermination s'est effectuée par analyse du bilan azoté dans les extraits urinaires. Ils améliorent le trophicité musculaire on a également mis en évidence le fait que ces courants stimulent les muscles striés des sphincters. Cette détermination s'est effectuée par des tests d'urodynamique et associés. Ces courants permettent le contrôle de la continence et la rééducation des incontinents.

Dans le cas particulier des cellules veineuses, le passage de ces courants qui permettent le dégagement de produit fibrinolytique et empêchent la thrombogénèse, renforce et préserve l'enduit protecteur à l'interieur de la paroi veineuse et active la fibrinolyse.

Un autre avantage thérapeutique de ces courants réside dans leur action antalgique qui se traduit par le drainage du système vasculaire éliminant les gonflements, les oedèmes et les stases qui sont à la source de certaines douleurs. De plus, ils favorisent la production d'endorphine - (hormone antidouleur), tout en diminuant le volume des membres traités.

Il résulte de ces divers avantages une meilleure nutrition des cellules, un meilleur trophisme, un meilleur métabolisme, une élimination de la stase veineuse, une élimination de la cellulite due à la meilleure vascularisation et au meilleur métaboslisme des cellules. On a également mis en évidence que ces courants agissent sur les valvules du tissu vasculaire, augmentent la turbidité et favorisent une bonne homogénéité des constituants du sang.

L'effet de ces courants est mémorisé par les cellules des muscles lisses et striés, ce qui leur donne une action durable. La mesure de résistivité de l'appareil permet de juger des progrès vasculaires du patient au cours du traitement et d'adapter ainsi la durée et l'intensité du traitement. Cette mesure de résistivité est la boucle préférentielle qui permet de réguler en courant la stimulation du patient et évite de changer les réglages en cours de stimulation. Cette mesure est reproductible d'une séance à l'autre. La différence de résistivité est analysée en pourcent pour juger des effets du traitement.

De ce qui précède, on voit que l'invention a également pour objet une méthode d'utilisation de cet appareil pour le traitement des maladies liées directement ou indirectement au mauvais fonctionnement ou aux déficiences des systèmes vasculaires et neurovasculaires. Cette méthode d'utilisation s'étend à la prévention des phlébites, thromboses, thrombophlébites, embolies pulmonaires, à la réduction des oedèmes postopératoires, à la thérapie de l'état thrombophlébique, à la prévention du catabolisme musculaire chez les comateux, les paraplégiques, les hémiplégiques, les opérés, les paralysés, les alités, les patients sous plâtre, en décubitus, les patients de moyens ou de longs séjours, etc., à l'amélioration du tropbisme musculaire et vasculaire, à la prévention et à la thérapie des escarres des patients allongés et des vieillards, à la rééducation fonctionnelle et à la kinésithérapie, à la prévention des accidents musculaires chez les sportifs, et à leur rééducation subséquente. Cet appareil peut également être adapté à tout acte de chirurgie, notamment orthopédique viscérale, en particulier pendant les opérations sur le petit bassin, en cardiologie, en pneumologie, en obstétrique, en réanimation, en soins intensifs et en soins de longs séjours, ainsi qu'en traumatologie et analogue. En outre il permet de traiter les maladies ou déficiences liées à la continence anale ou vésicale.

L'appareil de l'invention trouve naturellement son utilisation à l'hôpital, au cabinet du médecin, dans les services de médicine sportive, chez les esthéticiennes, en hospitalisation ou soins à domicile, par des personnels paramédicaux et analogue.

L'appareil selon l'invention peut être également utilisé pour la mise en oeuvre d'un procédé d'exploration fonctionnelle des tissus vasculaires et vasculonerveux, procédé selon lequel on capte au niveau de la zone corporelle à explorer des données résistives, hémodynamiques et rhéologiques relatives au milieu physiologique pendant une période de temps donnée, et l'on stocke lesdites données ; on envoie au moyen de l'appareil de l'invention pendant une période de temps donnée égale à ou différente de la période de demps précitées un ou plusieurs trains d'impulsions dans la zone corporelle à étudier ; et on capte après ou pendant l'application desdites impulsions les caractéristiques résistives, hémodynamiques et rhéologiques des milieux physiologiques pendant une période de temps égale à la première période de temps de captage, après quoi on stocke les données obtenues et on les compare avec les premières données déjà stockées, obtenant ainsi une indication de l'état du tissu exploré.

Il est clair que l'invention n'est nullement limitée aux formes et aux modes de réalisation décrits ci-dessus, mais qu'elle englobe toutes les modifications et variantes à la portée de l'homme de l'art, issues du même principe de base. C'est ainsi que l'on peut associer au micro-ordinateur décrit ci-dessus, n'importe quel moyen de logique programmée ou de logique de contrôle, par exemple une ou plusieurs mémoires programmables PROM et/ou mémoires effaçables et reprogrammables REPROM, ou bien connecter le microprocesseur sur un microprocesseur maître.

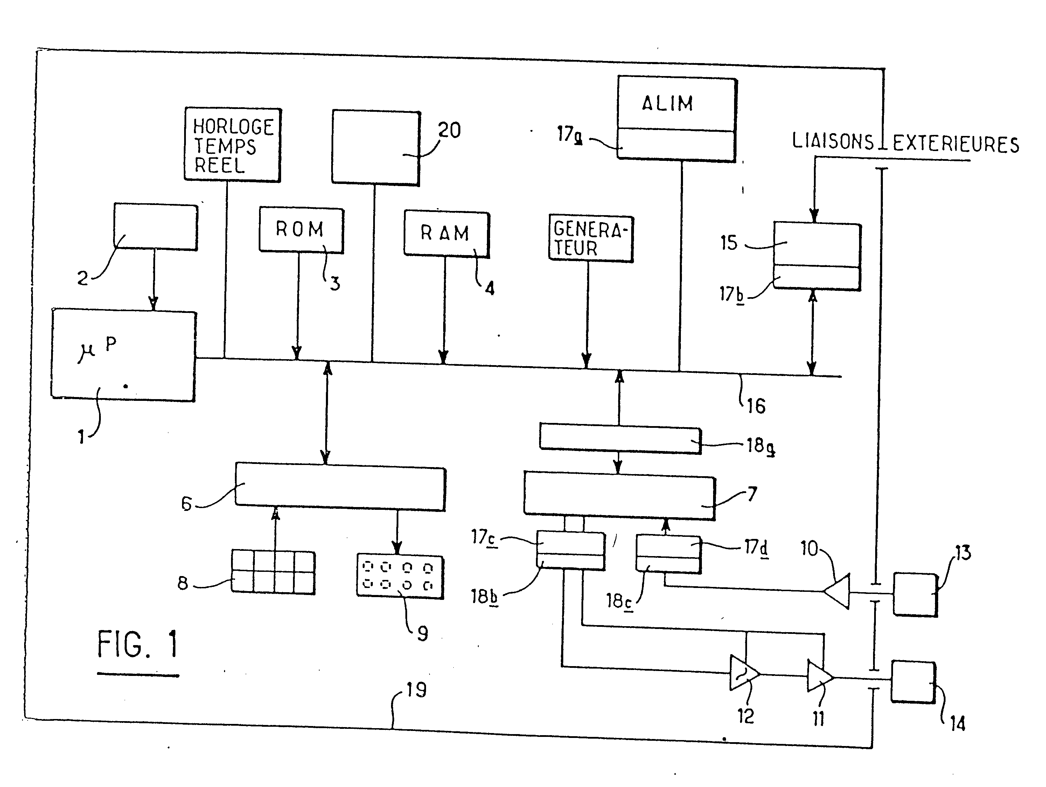

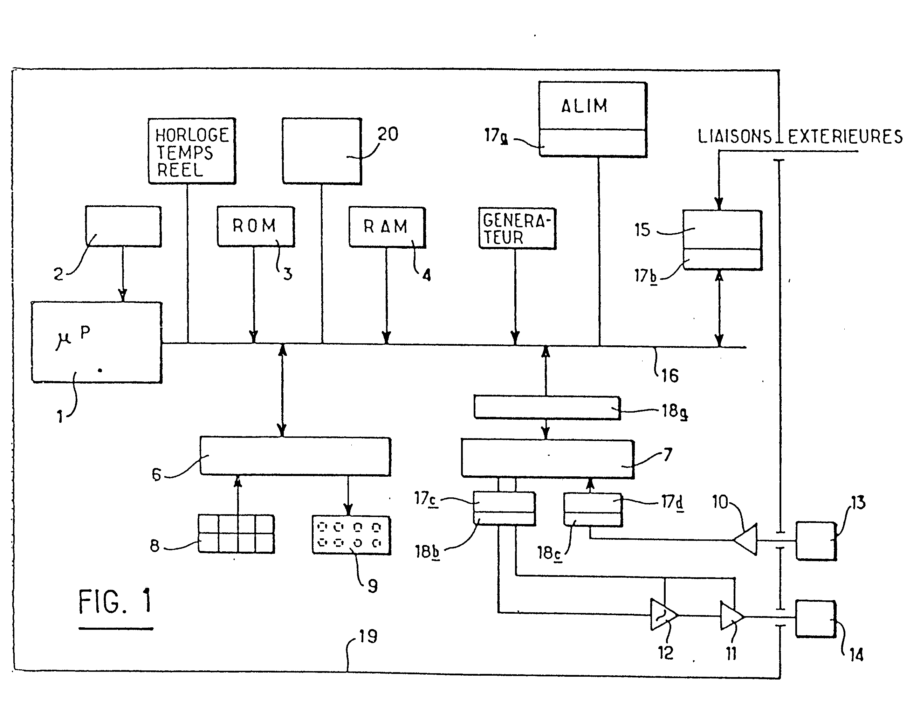

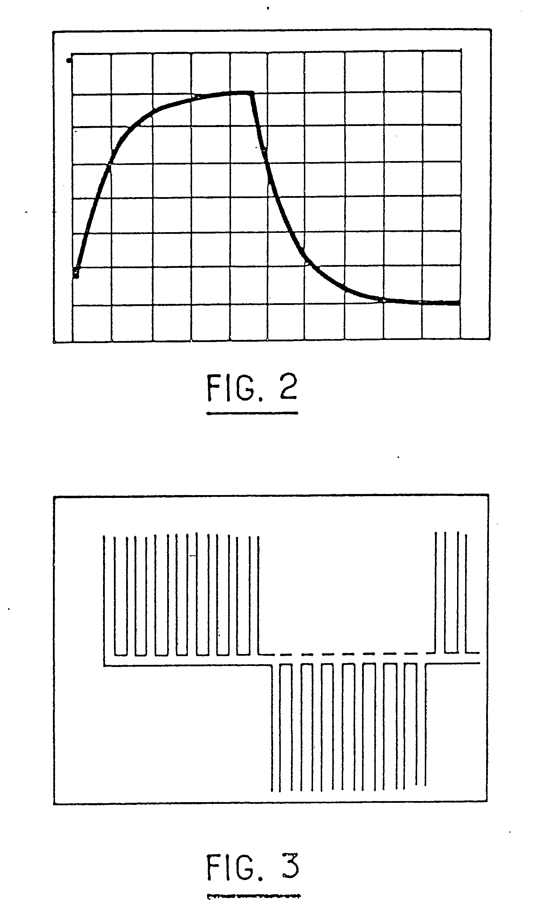

Par ailleurs, un microprocesseur maître, non représenté au dessin, peut être associé, dans le cadre de la présente invention avec un réseau de microprocesseurs programmables individuellement.

Une réalisation simplifiée peut mettre en oeuvre de la logique C-MOS.

Selon une autre forme de réalisation préférée, excluant les comparateurs, on peut mettre en oeuvre une boucle analysant le courant utilisé lors de la stimulation et coopérant avec un système logique analogique instituant des consignes de courant et permettant de faire varier la tension des impulsions de sortie pour maintenir ce courant constant en fonction du changement de résistivité du patient. La mesure du pourcentage de la variation de la résistivité indiquant la résistivité restante permet l'étude quantitative des effets de l'appareil sur le patient lors de son application et autorise le diagnostic. On notera également que les différents moyens et circuits mis en oeuvre dans l'invention peuvent être intégrés.

Ainsi se trouve résolu le problème de la thérapie des thrombophlébites, du catabolisme musculaire et de l'incontinence vésicale ou anale et des phénomènes associés à un mauvais drainage vasculaire, grâce à l'appareil de l'invention que génère des impulsions non douloureuses contrôlées par une boucle résistive autorisant l'exploration fonctionnelle et stimulant la totalité des muscles d'un ou de deux membres par la seule application de deux électrodes provoquant une accélération de la chasse veineuse. Cet appareil est avantageusement alimenté en courant continu, de préférence par batterie.

**Revendications**

1. Appareil électronique à programmation, notamment destiné à l'acquisition de l'information relative à la contraction des muscles, incluant notamment les muscles lisses vasomoteurs, par exemple les fibres musculaires lisses présentes dans le tissu vasculaire, à l'état hémodynamique à la stimulation du retour veineux et à la stimulation des sphincters anal et urétral, caractérisé par le fait qu'il comporte des moyens permettant de générer des impulsions progressives positives unidirectionnelles, progressives négatives unidirectionnelles, ou successivement positives et négatives par trains comportant une pluralité d'impulsions alternées, ces moyens générateurs comportant, disposés de façon logique, des moyens de programmation de mise en oeuvre comportant un système de logique programmée et un système de logique de contrôle et de comparaison agencés en boucle logique, des moyens d'intégration définissant la forme du signal d'impulsion générée et des moyens d'amplification, et que ces moyens générateurs d'impulsions sont associés à au moins deux électrodes disposées en des endroits appropriés du corps, de façon à permettre aux impulsions de circuler d'au moins une première zone d'application des impulsions à au moins une seconde zone d'application d'impulsions.

2. Appareil électronique à programmation, destiné à l'acquisition de l'information relative à la contraction des muscles, notamment des muscles lisses, par exemple les fibres musculaires lisses présentes dans le tissu vasculaire, caractérisé par le fait qu'il comporte des moyens permettant de générer des impulsions progressives positives unidirectionnelles, progressives négatives unidirectionnelles ou successivement positives et négatives par trains comportant une pluralité d'impulsions alternées, ces moyens générateurs comportant, disposés de façon logique, des moyens de programmation de mise en oeuvre comportant un sysptème de logique programmée et un système de logique de contrôle, des moyens d'intégration définissant la forme du signal d'impulsion générée et des moyens d'amplification, et que les moyens générateurs sont associés à des électrodes disposées en des endroits appropriés des membres inférieurs du sujet à traiter de façon à permettre aux impulsions de circuler alternativement d'un membre inférieur à l'autre en passant par le petit bassin ou d'un point quelconque du corps à un autre, ces divers moyens étant agencés en boucle logique.

3. Appareil selon la revendication 1 ou 2, caractérisé par le fait que les moyens de programmation sont constitués par au moins un ensemble mécanique/électronique logique-analogique constitué par au moins un microprocesseur associé à au moins une mémoire choisie parmi les mémoires ROM, PROM, EPROM, REPROM, les mémoires à bulle, les mémoires biologiques, les mémoires otpiques, les mémoires magnétiques et à au moins une mémoire vive RAM.

4. Appareil selon la revendication 1 ou 2, caractérisé en ce qu'il comporte, associés aux moyens de programmation, des moyens de captage permettant de détecter les paramètres indicateurs de l'état des tissus musculaires et/ou vasculaires, ainsi que de leur environnement, ces moyens de captage étant associés à des moyens capables, par filtrage et amplification, de transformer ces données paramétriques en signaux exploitables par les moyens de programmation.

5. Appareil selon la revendication 1 ou 2, caractérisé en ce qu'il comporte une boucle logique analysant et le signal d'impulsion généré et coopérant avec un système logique-analogique instituant des consignes de courant et permettant de faire varier la tension des impulsions de sortie pour maintenir ce courant constant en fonction du changement de résistivité du patient.

6. Appareil selon la revendication 3, caractérisé en ce que chaque appareil est associé à un réseau de distribution et d'information.

7. Méthode pour le traitement des maladies liées directement ou indirectement au mauvais fonctionnement ou aux déficiences des systèmes vasculaires, neurovasculaires, trophiques, cellulaires et d'incontinence, caractérisé en ce que l'on utilise un appareil selon l'une des revendications 1 à 6.

8. Procédé d'exploration fonctionnelle des tissus vasculaires, neurovasculaires, trophiques et cellulaires, mettant en oeuvre l'appareil selon l'une des revendications 1 à 7, caractérisé en ce que l'on capte, au niveau d'une zone corporelle prédéterminée, les données résistives, hémodynamiques et rhéologiques relatives au milieux physiologiques pendant une période de temps donnée, les données obtenues étant stockées, que l'on envoie dans ladite zone corporelle un ou plusieurs trains d'impulsions progressives positives unidirectionnelles, progressives négatives unidirectionnelles, ou successivement positives et négatives par train comportant une pluralité d'impulsions alternées, pendant une période de temps prédéterminée, égale à ou différente à la première période de temps de captage des données résistives, hémodynamiques et rhéologiques, et que l'on capte après ou pendant l'application desdites impulsions, les caractéristiques résistives, hémodynamiques et rhéologiques des milieux physiologiques pendant une période de temps égale à la première période de temps de captage, après quoi l'on stocke les données obtenues et on les compare avec les premières données préalablement sotckées, obtenant ainsi une indication de l'état des tissus explorés.

9. Appareil selon l'une des revendications 1 à 5, caractérisé en ce que les courants ou signaux utilisés présentent les caractéristiques suivantes :

-une constante de temps égale ou de l'ordre de 40 $\mu s \pm 20~\mu s$;

-une durée d'extinction égale à la durée de l'établissement de l'impulsion ;

-une largeur à la base égale à 3 ms ± 30 ms ;

-une amplitude de crête réglable de 0 à 130 volts et de -130 à 0 V ;

-un courant de 0,05 mA à 2 mA ;

-une période égale à 541 ms ± 200 ms.

10. Appareil selon la revendication 9, caractérisé en ce que la durée de l'impulsion ne dépasse jamais 80 % de la période d'impulsion, tandis que la tension de crête ne dépasse à aucun moment 125 % de la valeur de réglage, les variations de la tension de sortie ne produisant aucune modification de la fréquence de réglage, et que dans le cas de trains d'impulsions progressives alternées, il est préférable de mettre en oeuvre des terrains de 8 impulsions (-6, + 30) et dont la période de récurrence se situe entre -8 et + 20 s, par exemple égale à 10 s.

FIG. 1

HORLOGE TEMPS REEL

ALIM

LIAISONS EXTERIEURES

ROM

RAM

GENERA-TEUR

μP

0 202 160

0 202 160

FIG. 1

FIG. 2

FIG. 3